# EUROPEAN PATENT APPLICATION

(11) **EP 2 636 394 A1**
(43) Date of publication of application: **11.09.2013**
(21) Application number: 11838016.1
(22) Date of filing: 01.11.2011
(51) Int. Cl.: A61F 13/15, A61F 13/49

(54) **DISPOSABLE DIAPER**

(30) Priority: 31.10.2011 JP 2011238979; 02.11.2010 JP 2010246695
(71) Applicant: Kao Corporation, Chuo-Ku Tokyo 103-8210 (JP)
(72) Inventor: KIKKAWA, Muneyoshi, Haga-gun Tochigi 321-3497 (JP); SATO, Nobuya, Haga-gun Tochigi 321-3497 (JP)
(74) Representative: Vossius & Partner
(86) International application number: PCT/JP2011/075162
(87) International publication number: WO 2012/060366

(57) **Abstract**

A disposable diaper (1A) of the present invention is provided with an absorbent body (5) which includes a topsheet (2), a backsheet (3) and an absorbent member (4) as being sectioned into a front section (A), a rear section (B) and a crotch section (C). Here, the absorbent member (4) includes a laminated absorbent member (41) which is formed by sandwiching high-absorbent polymer with nonwoven (411, 412) and which is arranged over a range from the front section (A) to the rear section (B). The absorbent member (4) and the topsheet (2) are not fixed at the rear section (B) and are fixed at the front section (A) and the crotch section (C). The absorbent body (5) includes elastic members (9) across the absorbent member (4) in a stretching state between the absorbent member (4) and the topsheet (2) at the rear section (B). The elastic members (9) are arranged with both end portions (9s) thereof fixed between the topsheet (2) and the backsheet (3), so that the absorbent member (4) and the topsheet (2) are separated from each other while used owing to contraction of the elastic members (9).

## Description

### Technical Field

The present invention relates to a disposable diaper such as an open-style disposable diaper.

### Background Art

A variety of disposable diapers in which a top sheet and an absorbent member are intimately fixed have been proposed to increase absorbing speed of absorbing body fluid. However, when the top sheet and the absorbent member is intimately contacted, there may be a case that body fluid once absorbed by the absorbent member returns to the topsheet while increasing absorbing speed of body fluid.

By the way, the present applicant proposed a disposable diaper in which a waist gather is arranged at a periphery of a waist opening portion and a torso gather is arranged separately in parallel to the waist gather (see Patent Literature 1). The diaper described in Patent Literature 1 provides excellent fit to wearer's waist. However, since a topsheet and an absorbent member are not intimately contacted, there is room for improvement in speed for absorbing body fluid.

Further, Patent Literature 2 discloses a disposable diaper which includes an elastic band with an elastic member arranged between a base layer and a top face layer. However, in Patent Literature 2, there is not description regarding intimate contact between the topsheet and an absorbent member to increase absorbing speed of absorbing body fluid. Further, in Patent Literature 2, there is no description regarding arrangement of the elastic band to separate the absorbent member and the topsheet in distance.

Patent Literature 1: JP8-71103A
Patent Literature 2: WO2009-145860

### Summary of Invention

The present invention relates to a disposable diaper capable of solving the abovementioned drawbacks in the related art.

The present invention provides a disposable diaper including an elongated absorbent body which includes a liquid permeable topsheet, a liquid low-permeable backsheet, and an absorbent member arranged between the sheets, and is sectioned into a front section, a rear section, and a crotch section. The absorbent member includes a laminated absorbent member which is formed by sandwiching high-absorbent polymer with two sheets of nonwoven and which is arranged over a range from the front section to the rear section. The absorbent member and the topsheet are not fixed at the rear section and are fixed at the front section and the crotch section. The absorbent body includes a plurality of elastic members across the absorbent member in a stretching state between the absorbent member and the topsheet at the rear section. The plurality of elastic members is arranged as being mutually spaced in the longitudinal direction while both end portions of each elastic member are fixed between the topsheet and the backsheet, so that the absorbent member and the topsheet are separated from each other owing to contraction of the plurality of elastic members while used.

### Brief Description of the Drawings

[FIG. 1] FIG. 1 is a partially-sectioned plane view illustrating a state that an open-style disposable diaper of a first embodiment of the present invention is stretched and opened.
[FIG. 2] FIG. 2 is a sectional view at line Y1-Y1 of FIG. 1.
[FIG. 3] FIG. 3 is a sectional view at line X1-X1 of FIG. 1 while used.
[FIG 4] FIG. 4 is a plane view of an elastic structure included in the disposable diaper illustrated in FIG. 1.
[FIG 5] FIG. 5 is a partial sectional view at line Y1-Y1 of FIG. 1 while used.
[FIG 6] FIG. 6 is a partially-sectioned plane view illustrating a state that an open-style disposable diaper of a second embodiment of the present invention is stretched and opened.
[FIG. 7] FIG. 7 is a sectional view at line X2-X2 of FIG. 6 while used.
[FIG 8] FIG. 8 is a plane view of an elastic structure of another embodiment.
[FIG. 9] FIG. 9 is a plane view illustrating a state that an open-style disposable diaper of a third embodiment of the present invention is stretched and opened.

### Description of Embodiments

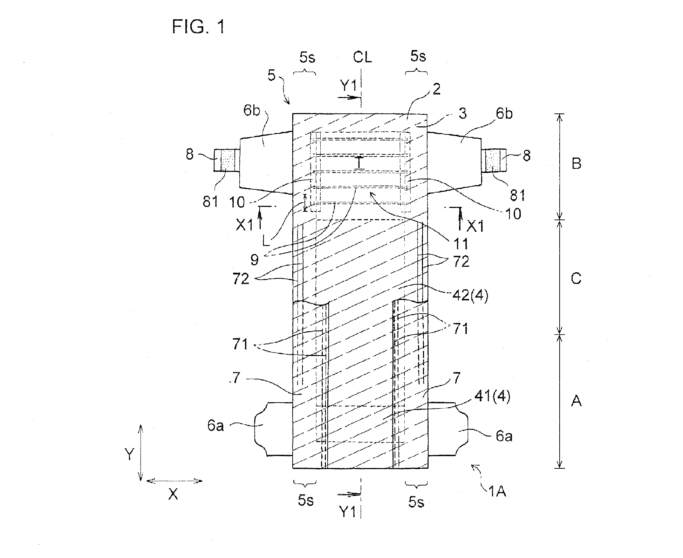

In the following, a disposable diaper of the present invention will be described based on a preferable first embodiment with reference to FIGs. 1 to 5.

As illustrated in FIG. 1, a disposable diaper 1A (hereinafter, also called a diaper 1A) of a first embodiment being a so-called open-style disposable diaper is provided with an elongated absorbent body 5 which includes a liquid permeable topsheet 2, a liquid low-permeable backsheet 3, and an absorbent member 4 arranged between the sheets 2, 3, and which is sectioned into a front section A, a rear section B, and a crotch section C. As illustrated in FIG. 1, the diaper 1A is formed as being bilaterally symmetric against a center line CL extending in a longitudinal direction of the diaper 1A. Here, a Y-direction indicated in each drawing denotes a direction which is in parallel to the center line CL, and the same direction as the diaper longitudinal direction. Further, an X-direction indicated in each drawing denotes a direction which is perpendicular to the center line CL, and the same direction as the diaper width direction.
Further, in the present specification, a skin-facing face denotes a face arranged to a skin side of a wearer while worn out of front and back faces of each member such as the absorbent body 5. A non-skin-facing face denotes a face facing to a side opposite to the skin side of the wearer while worn out of the front and back faces of each member such as the absorbent body 5.

Specifically, as illustrated in FIG. 1, the diaper 1A is provided with the absorbent body 5 which includes the front section A adapted to be worn about the front of a wearer, the rear section B adapted to be worn about the back of a wearer, and the crotch section C adapted to be worn between the front section A and the rear section B. The absorbent body 5 is rectangle-shaped in a state of being opened and extended. As illustrated in FIG. 1, the front section A, the rear section B, and the crotch section C denote three sections obtained by sectioning to approximately trisect the entire length of the absorbent body 5 in the longitudinal direction (Y-direction) in a state of being opened and extended. As illustrated in FIG. 1, the absorbent body 5 includes the topsheet 2 which forms an inner face of the diaper 1A, the backsheet 3 which forms an outer face of the diaper 1A, and the liquid-retaining absorbent member 4 which is interposed between the sheets 2, 3. As illustrated in FIG. 1, the topsheet 2 and the backsheet 3 cover the entire face at the skin-facing face side of the absorbent member 4 and the entire face at the non-skin-facing face side of the absorbent member 4 respectively and are extended outward from a periphery of the absorbent member 4. A section of the topsheet 2 and a section of the backsheet 3 which are outwardly-extended from the periphery of the absorbent member 4 are fixed as illustrated in FIG. 1. Further, as illustrated in FIG. 1, a length of the topsheet 2 in the X-direction is shorter than a length of the backsheet 3 in the X-direction.

As illustrated in FIG. 1, a side sheet 7 is arranged at each of both side portions 5s, 5s in the Y-direction of the absorbent body 5 of the diaper 1A via the topsheet 2. As illustrated in FIG. 1, the side sheet 7 is arranged and fixed over the entire range of the side portion in the Y-direction at the skin-facing face side of the topsheet 2. As illustrated in FIG. 1, an end portion of each side sheet 7 at the inner side (being close to the center line CL) in the X-direction is a free end. An elastic member 71 for forming a standing gather is arranged and fixed to a vicinity of the free end in a state of being stretched in the longitudinal direction (Y-direction). A section having a predetermined width from the free end is separated from the topsheet 2 while worn owing to a contraction force of the elastic member, so that the standing gather is formed.

As illustrated in FIG. 1, an end portion of each side sheet 7 at the outer side in the X-direction is fixed to the backsheet 3. An elastic member 72 for forming a leg gather is arranged and fixed at the crotch section C between the end portion of each side sheet 7 at the outer side and the backsheet 3 in a state of being stretched in the longitudinal direction (Y-direction). The leg gather is formed with a contraction force of the elastic member while worn.

Further, as illustrated in FIG. 1, the diaper 1A includes a right-left pair of stretchable panels 6b, 6b arranged at both bilateral outer sides of the rear section B of the absorbent body 5. End portions being close to the center line CL of the pair of stretchable panels 6b, 6b of the diaper 1A are fixed to the non-skin-facing face of the backsheet 3 at the rear section B and are continuously arranged bilaterally outward from the rear section B of the absorbent body 5. Here, in the diaper 1A, the pair of stretchable panels 6b, 6b is fixed to the non-skin-facing face of the backsheet 3. However, it is also possible to be fixed between the backsheet 3 and each side sheet 7 arranged via the topsheet 2. A fastening tape 8 to which a fastening portion 81 formed of a male member of a mechanical hook-and-loop fastener is attached is attached to each stretchable panel 6b as being extended outward in the X-direction. The stretchable panel 6b is fixed using means such as adhesive and fusion.

Further, as illustrated in FIG. 1, the diaper 1A includes a right-left pair of panel members 6a, 6a arranged at both bilateral outer sides of the front section A of the absorbent body 5. As illustrated in FIG. 1, end portions being close to the center line CL of the pair of panel members 6a, 6a of the diaper 1A are fixed between the backsheet 3 and each side sheet 7 arranged via the topsheet 2 at the front section A and are continuously arranged bilaterally outward from the front section A of the absorbent body 5. Here, in the diaper 1A. the pair of panel members 6a, 6a is fixed between the backsheet 3 and each side sheet 7. However, it is also possible to be fixed to the non-skin-facing face of the backsheet 3. The panel member 6a is fixed using means such as adhesive and fusion.

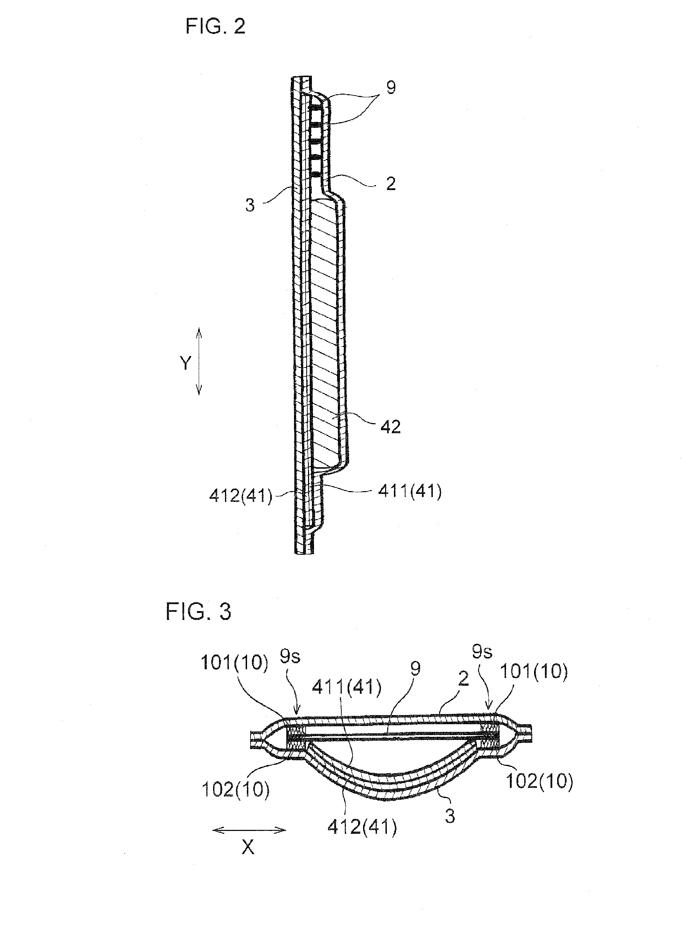

As illustrated in FIG. 2, the absorbent member 4 includes a laminated absorbent member 41 which is formed by sandwiching high-absorbent polymer (not illustrated) with two sheets of nonwoven 411, 412 and includes a sublayer 42 which is fixed on the laminated absorbent member 41 located at the crotch section C of the diaper 1A. As illustrated in FIG. 1, the laminated absorbent member 41 is arranged over a range from the front section A to the rear section B. As illustrated in FIG. 1, the laminated absorbent member 41 is rectangle-shaped in plane view. As illustrated in FIGs. 1 and 2, the two sheets of nonwoven 411, 412 have the same shape and size and have a profile being matched with a profile of the laminated absorbent member 41.

A basis weight of the laminated absorbent member 41 is appropriately set in accordance with usage application and the like of a disposable diaper to which the laminated absorbent member 41 is assembled. For example, in a case that the diaper 1A is a diaper for an infant (young month-old infant), it is preferable that the basis weight of the laminated absorbent member 41 is in a range of 80 to 400 g/m² from a viewpoint of balance among thinning, flexibility, and liquid absorbability. The laminated absorbent member 41 is formed by joining the two sheets of nonwoven 411, 412 in a state that high absorbent polymer (not illustrated) is sandwiched between the two sheets of nonwoven 411, 412 with joining means (adhesive) such as hot-melt type adhesive.

It is preferable that content of high absorbent polymer (not illustrated) in the laminated absorbent member 41 is 95% or more by mass against the total mass of the laminated absorbent member 41. Various types traditionally used in the technical field may be used without specific limitations as the high absorbent polymer (not illustrated). Examples thereof include sodium polyacrylate, (acrylic acid-vinyl alcohol) copolymer, crosslinked sodium polyacrylate, (starch-acrylic acid) graft polymer, (isobutylene-maleic acid anhydride) copolymer and saponification thereof, kalium polyacrylate, and cesium polyacrylate. Here, one type may be used solely or two or more types may be used as being mixed. Either of a particle type and a fiber type may be used as the absorbent polymer.

It is preferable that a basis weight of the two sheets of nonwoven 411, 412 of the laminated absorbent member 41 is in a range of 7 to 30 g/m². Although the two sheets of nonwoven 411, 412 have the same shape and size and have the same basis weight in the diaper 1A, they may be different as well. Examples of the two sheets of nonwoven 411, 412 include nonwoven such as spunbond nonwoven, meltblown nonwoven, thermalbonded nonwoven, needle-punched nonwoven, spanlaced nonwoven and airlaid nonwoven, and a combined sheet with two types or more off the above laminated. The diaper 1A adopts spunbond-meltblown-spunbond (SMS) nonwoven being nonwoven integrated in a state that one meltblow nonwoven layer is placed between two spunbond nonwoven layers.

As illustrated in FIG. 1, the sublayer 42 provided to the absorbent member 4 of the diaper 1A is rectangle-shaped in plane view. As illustrated in FIG. 2, a length in the width direction (X-direction) is the same as a length of the laminated absorbent member 41 in the width direction (X-direction) and a length in the longitudinal direction (Y-direction) is shorter than a length of the laminated absorbent member 41 in the longitudinal direction (Y-direction). From a viewpoint to suppress a phenomenon (wet-back) that absorbed body fluid returns to the topsheet 2 side by reinforcing body fluid absorbability at the crotch section C, an area of the sublayer 42 is equal to or less than the total area of the skin-facing face of the laminated absorbent member 41 preferably, and is in a range of 30% to 50% more preferably. As illustrated in FIG. 1, the sublayer 42 is arranged and fixed onto the skin-facing face of the laminated absorbent member 41 and is extended from the crotch section C toward the front section A in the diaper 1A.

It is preferable that a basis weight of the sublayer 42 is in a range of 40 to 300 g/m². A sheet which is structured mainly with hydrophilic fibers (preferably, a sheet with content of the hydrophilic fibers being 90% or more by mass) may be adopted as the sublayer 42. Examples of the sheet include paper, nonwoven, and a web. Fibers having hydrophilic surfaces may be used as the hydrophilic fibers without specific limitations as long as being capable of forming a sheet with a high degree of freedom of mutual fibers in a wet state. In addition to cellulose fibers, hydrophilic synthetic fibers, and synthetic fibers on which a hydrophilicity process is performed, examples of the hydrophilic fibers include denatured (cellulose) fibers disclosed in JP2010-526632A exemplifying kneaded and/or curled (curly) chemically-stiffened (cellulose) fibers, and kneaded and/or curled chemically-stiffened bridged cellulose or synthetic polymer fibers. Here, one type may be used solely or two or more types may be used as being mixed. Here, in addition to the hydrophilic fibers, other constituents such as absorbent polymer and deodorant may be included in the sublayer 42.

In the present invention, the absorbent member 4 and the topsheet 2 are not fixed at the rear section B and are fixed at the front section A and the crotch section C, as illustrated in FIG. 1. Specifically, in the diaper 1A, the absorbent member 4 is structured with the laminated absorbent member 41 which is arranged from the rear section B to the front section A and the sublayer 42 which is arranged and fixed onto the skin-facing face of the laminated absorbent member 41 as being extended from the crotch section C toward the front section A. Here, fixing is performed in all sections except for that the skin-facing face of the laminated absorbent member 41 located at the rear section B and the non-skin-facing face of the topsheet 2 are not fixed. That is, the skin-facing face of the sublayer 42 located at the crotch section C and the non-skin-facing face of the topsheet 2 are fixed and the skin-facing face of each of the sublayer 42 and the laminated absorbent member 41 located at the front section A and the non-skin-facing face of the topsheet 2 are fixed.

The absorbent member 4 and the topsheet 2 are fixed using hot-melt adhesive. Examples of the hot-melt adhesive include block-copolymer-based hot-melt adhesive such as styrene-isoprene-styrene block copolymer (SIS), styrene-hutadiene-styrene block copolymer (SBS), and styrene-ethylene-butylene-styrene copolymer (SEBS). Examples of a method of applying hot-melt adhesive include an applying method in a spiral fashion and an applying method in a bead fashion. In the diaper 1A, hot-melt adhesive is applying in a spiral fashion. In a case of being applied in a spiral fashion, hot-melt adhesive is applied in a range of 2 to 20 g/m² preferably, and in a range of 3 to 10 g/m² more preferably. Here, the abovementioned fixing between the section of the topsheet 2 and the section of the backsheet 3 which are outwardly-extended from the periphery of the absorbent member 4 may be performed similarly to the fixing between the absorbent member 4 and the topsheet 2.

As illustrated in FIG. 1, in the absorbent body 5 of the disposable diaper of the present invention, a plurality of stretched elastic members 9 across the absorbent member 4 is arranged between the absorbent member 4 and the topsheet 2 at the rear section B. The plurality of elastic members 9 is arranged as being mutually spaced in the longitudinal direction (Y-direction). Specifically, it is preferable that the number of the elastic members 9 arranged at the rear section B is in a range of 3 to 15. The number in the diaper 1A is 5. The respective elastic members 9 are stretched in the X-direction. In the diaper I A, the respective elastic members 9 are arranged across the laminated absorbent member 41 of the absorbent member 4 located at the rear section B. Further, in the diaper 1A, the respective elastic members 9 are arranged as being approximately equally spaced in the longitudinal direction (Y-direction). Although the respective elastic members 9 in the diaper 1A are arranged as being approximately equally spaced in the longitudinal direction (Y-direction), it is also possible to be arranged without being equally spaced.

Examples of the elastic member 9 include natural rubber (or synthetic rubber) and polyurethane spandex elastic fibers. When natural rubber (synthetic rubber) is used as the elastic member 9, an elastic member with a thickness being in range of 0.05 to 3 mm and a width being in a range of 0.2 to 5 mm is to be adopted. Stress of a single yam of such natural rubber (synthetic rubber) at the time of being stretched by 100% is in a range of 1 to 70 gf preferably, and in a range of 1 to 30 gf more preferably. Single yarns of natural rubber (synthetic rubber) having the above stress are arranged to have a stretching ratio being 100% or larger preferably, and being 200% or larger more preferably. Here, the stretching ratio denotes a ratio of an increased length with stretching against a natural length. For example, when a length being 10 cm is stretched to a length being 20 cm, the stretching ration is 100%.

When polyurethane spandex elastic fibers are used as the elastic member 9, a single yam size may be in a range of 10 to 3360 deniers preferably, and in a range of 70 to 1120 deniers more preferably. Denier is a unit indicating thickness of a yarn. A yam having 1 g with 9000 m is called 1 denier. Such spandex elastic fibers are arranged to have a stretching ratio being 30% or larger preferably, and 100% or larger more preferably.

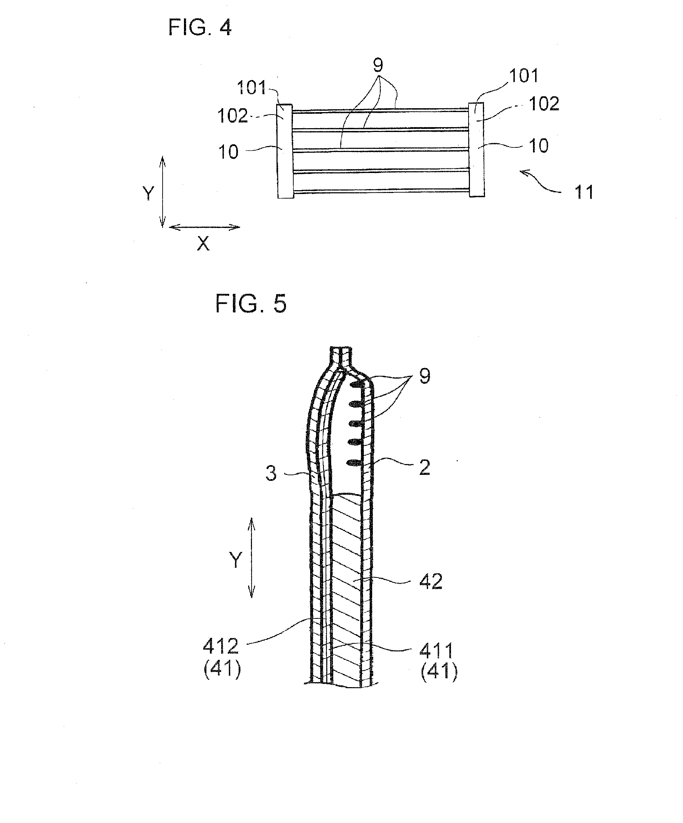

As illustrated in FIG. 1, each of the plurality of elastic members 9 in the disposable diaper of the present invention is arranged while both end portions 9s, 9s of the elastic member 9 are fixed between the topsheet 2 and the backsheet 3, so that the absorbent member 4 and the topsheet 2 are separated from each other owing to contraction of the plurality of elastic members 9 while used. In the diaper 1A, both the end portions 9s, 9s of each of the plurality of elastic members 9 are fixed with a pair of panel members 10, 10 extending in the longitudinal direction (Y-direction) as illustrated in FIG. 1. Both the end portions 9s of the elastic members 9 are fixed between the topsheet 2 and the backsheet 3 via the pair of panel members 10, 10. Specifically, each of the pair of panel members 10, 10 is rectangle-shaped as being elongated in the Y-direction in plane view, as illustrated in FIG. 1 and includes two sheet pieces 101, 102 having the same shape and size, as illustrated in FIG. 3. The two sheet pieces are joined with joining means (adhesive) such as hot-melt type adhesive in a state that both the end portions 9s, 9s of each of the elastic member 9 are sandwiched respectively by the two sheet pieces as illustrated in FIG. 3. Accordingly, a ladder-shaped elastic structure 11 in which the plurality of elastic members 9 is arranged between the pair of panel members 10, two as being spaced in the longitudinal direction (Y-direction) is formed, as illustrated in FIG. 4. The elastic structure 11 is arranged between the absorbent member 4 and the topsheet 2 in the thickness direction, as illustrated in FIG. 1. Specifically, regarding a configuration of the elastic structure 11, the pair of panel members 10, 10 is fixed between the topsheet 2 and the backsheet 3 and the respective elastic members 9 are not fixed to the absorbent member 4, as illustrated in FIGs. 1 and 3. That is, as illustrated in FIG. 3, both the end portions 9s, 9s of each of the elastic members 9 are fixed respectively at the outer side in the X-direction from a side edge of the absorbent member 4 along the longitudinal direction (Y-direction). More specifically, as illustrated in FIG. 3, the end portion 9s of each elastic member 9 is fixed at the outer side in the X-direction from the side edge of the laminated absorbent member 41 structuring the absorbent member 4 along the longitudinal direction (Y-direction).

As illustrated in FIG. 1, in the diaper 1A, the plurality of elastic members 9 is arranged as being spaced in the longitudinal direction (Y-direction) with a part thereof located beyond a section T which is sandwiched by the pair of stretchable panels 6b, 6b. Specifically, the ladder-shaped elastic structure 11 formed as described above is extended toward the crotch section C side beyond the section T which is sandwiched by the pair of stretchable panels 6b, 6b only by a section L illustrated in FIG. 1. The plurality of elastic members 9 located at the section L is arranged as being spaced in the longitudinal direction (Y-direction). As illustrated in FIG. 2, the ladder-shaped elastic structure 11 is arranged on the skin-facing face of the laminated absorbent member 41 of the absorbent member 4 located at the rear section B. The pair of panel members 10, 10 of the elastic structure 11 is arranged and fixed respectively between the topsheet 2 and the backsheet 3. Accordingly, both the end portions 9s, 9s of each elastic member 9 are fixed respectively between the topsheet 2 and the backsheet 3 via the panel members 10.

Since the disposable diaper of the present invention includes the plurality of elastic members 9 arranged in a stretching state as described above, the absorbent member 4 and the topsheet 2 are separated owing to contraction of the plurality of elastic members 9 when used, as illustrated in FIGs. 3 and 5. Specifically, since the skin-facing face of the laminated absorbent member 41 located at the rear section B and the non-skin-facing face of the topsheet 2 are not fixed and the plurality of elastic members 9 in a stretching state as being stretched in the X-direction is contracted while used, the laminated absorbent member 41 located at the rear section B and the topsheet 2 are separated and space is to be formed therebetween, as illustrated in FIGs. 3 and 5.

Materials for forming the diaper 1A of the present embodiment will be described.
Anything may be adopted without specific limitations as the topsheet 2 and the backsheet 3 as long as being normally used respectively for an absorbent article such as a disposable diaper. For example, the topsheet 2 may adopt hydrophilic and liquid permeable non-woven or the like and the backsheet 3 may adopt a liquid impermeable or water-repellent resin film, a laminated member of the resin film and nonwoven, or the like. Anything may be adopted without specific limitations as the side sheet 7 which forms the standing gather and the leg gather as long as being normally used respectively for an absorbent article such as a disposable diaper. Here, a stretchable film, nonwoven, woven, a laminated sheet thereof, or the like may be used. The elastic member 71 for forming the standing gather and the elastic member 72 for forming the leg gather may adopt thread-like elastic materials formed of natural rubber, polyurethane, polystyrene-polyisoprene copolymer, polystyrene-polybutadiene copolymer, or polyethylene-α-olefin copolymer such as ethyl-acrylate-ethylene.

The sheet pieces 101, 102 forming the elastic structure 11 may adopt paper or nonwoven such as spunbond nonwoven, meltblown nonwoven, thermalbonded nonwoven, needle-punched nonwoven, spanlaced nonwoven, and airlaid nonwoven.

Anything may be adopted without specific limitations as the elastic member 71 for forming the standing gather and the elastic member 72 for forming the leg gather as long as being normally used respectively for an absorbent article such as a disposable diaper. For example, it is possible to adopt thread-like elastic materials formed of natural rubber, polyurethane, polystyrene-polyisoprene copolymer, polystyrene-polybutadiene copolymer, or polyethylene-α-olefin copolymer such as ethyl-acrylate-ethylene.

For examples, the stretchable panel 6b may adopt (1) a sheet with a stretchable fiber layer integrated to both faces or one face of an elastic fiber layer, (2) a sheet with a stretchable fiber layer integrated to both faces or one face of a net-shaped elastic sheet, or (3) a sheet with a stretchable fiber layer integrated to both faces or one face of an elastic sheet made of an elastic film. Anything may be adopted without specific limitations as the panel member 6a as long as being normally used for an absorbent article such as a disposable diaper. For example, it is possible to adopt liquid impermeable nonwoven or the like. Anything may be adopted without specific limitations as the fastening tape 8 as long as being normally used for an absorbent article such as a disposable diaper. For example, as engaging projections of the fastening tape 8, it is possible to adopt a male member of "Magic Tape (a registered trademark)" (manufactured by Kuraray CO., Ltd), "Quicklon (a registered trademark)" (manufactured by YKK AP Inc.), "Magicross (a registered trademark)" (manufactured by Kanebo Bell-Touch, Ltd), or the like.

Fixing of the topsheet 2, the backsheet 3, the side sheet 7, and the fastening tape 8 is performed with hot-melt type adhesive or fusion means, such as heat emboss, ultrasonic emboss, and high-frequency emboss, which is normally used for an absorbent article such as a disposable diaper.

Next, operational effects obtained when using the abovementioned disposable diaper 1A of the first embodiment of the present invention will be described.
As illustrated in FIGs. 1 and 2, in the diaper 1A, the absorbent member 4 and the topsheet 2 are not fixed at the rear section B and are fixed at the front section A and the crotch section C. At the rear section B, the plurality of stretched elastic members 9 across the absorbent member 4 is arranged between the absorbent member 4 and the topsheet 2. Both the end portions 9s, 9s of each of the plurality of elastic members 9 are fixed between the topsheet 2 and the backsheet 3. Accordingly, as illustrated in FIGs. 3 and 5, the absorbent member 4 and the topsheet 2 are separated from each other owing to contraction of the plurality of elastic members 9 while used. According to the diaper 1A formed as described above, since the absorbent member 4 and the topsheet 2 are fixed with intimate contact particularly at the crotch section C, leading is continued from the topsheet 2 through the absorbent member 4. Consequently, absorbing speed of body fluid is increased. Here, even when body fluid absorbed by the absorbent member 4 spreads to the rear section B side, the body fluid once absorbed by the absorbent member is less likely to return owing to separation between the absorbent member 4 and the topsheet 2. Further, since the diaper 1A includes the sublayer 42 which is fixed onto the skin-facing face of the laminated absorbent member 41 located at the crotch section C, absorbing speed of body fluid at the crotch section C is further increased and returning of body fluid at the crotch section C can be suppressed.

Further, in the diaper 1A, both the end portions 9s, 9s of each of the plurality of elastic members 9 are fixed with the pair of panel members 10, 10 extending in the longitudinal direction (Y-direction) as illustrated in FIG. 1 to form the ladder-shaped elastic structure 11 (see FIG. 4). In the ladder-shaped elastic structure 11 (see FIG. 4) as described above, only the plurality of elastic members 9 is arranged between the pair of panel members 10, 10. Accordingly, body fluid spreading to the rear section B side is less likely to be maintained between the topsheet 2 and the plurality of elastic members 9 and steaming thereat is less likely to be caused.

Further, as illustrated in FIG. 1, in the diaper 1A. the plurality of elastic members 9 is extended toward the crotch section C side beyond the section T which is sandwiched by the pair of stretchable panels 6b. 6b only by the section L illustrated in FIG. 1. With the above, when used with the pair of stretchable panels 6b, 6b stretching outward in the X-direction, the plurality of elastic members 9 located in the section T sandwiched by the pair of stretchable panels 6b, 6b is stretched in the X-direction while the plurality of elastic members 9 located in the section L illustrated in FIG. 1 is hardly to be stretched in the X-direction. Accordingly, while using the diaper 1A, the absorbent member 4 and the topsheet 2 are separated owing to contraction of the plurality of elastic members 9 in the section L, so that body fluid spread to the rear section B side is less likely to return.

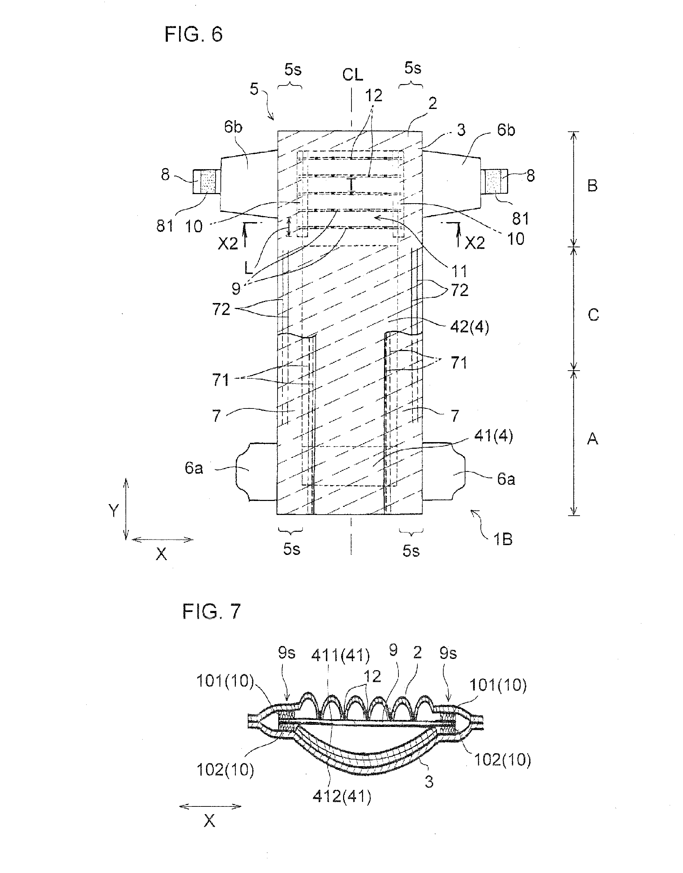

Next, a disposable diaper of a second embodiment of the present invention will be described based on FIGs. 6 and 7.
A disposable diaper 1B (hereinafter, also called a diaper 1B) of the second embodiment will be described mainly on points being different from the disposable diaper 1A of the first embodiment. Points which are not specifically described are similar to the diaper 1A and description of the diaper 1A is appropriately applied.

As illustrated in FIGs. 6 and 7, in the diaper 1B of the second embodiment, the plurality of elastic members 9 at center sections thereof is fixed to the topsheet 2 via an adhesive 12 which is discontinuously arranged in the width direction (X-direction) and the longitudinal direction (Y-direction). A gather is formed at the topsheet 2 owing to contraction of the plurality of elastic members 9 while used. In this manner, the topsheet 2 has an uneven shape due to the gather. In the diaper 1B being similar to the diaper 1A, the absorbent member 4 is structured with the laminated absorbent member 41 which is arranged from the rear section B to the front section A and the sublayer 42 which is arranged and fixed onto the skin-facing face of the laminated absorbent member 41 and which is extended from the crotch section C toward the front section A. Here, the skin-facing face of the laminated absorbent member 41 located at the rear section B and the non-skin-facing face of the topsheet 2 are not fixed, while the skin-facing face of the sublayer 42 located at the crotch section C and the non-skin-facing face of the topsheet 2 are fixed, and the skin-facing face of each of the sublayer 42 and the laminated absorbent member 41 located at the front section A are fixed to the non-skin-facing face of the topsheet 2.

Similarly to the diaper 1A, the diaper 1B includes the ladder-shaped elastic structure 11 (see FIG. 4) in which the plurality of elastic members 9 is arranged between the pair of panel members 10, 10 as being spaced in the longitudinal direction (Y-direction). As illustrated in FIG. 6, the ladder-shaped elastic structure 11 is arranged on the skin-facing face of the laminated absorbent member 41 of the absorbent member 4 located at the rear section B. The pair of panel members 10, 10 of the elastic structure 11 is arranged and fixed respectively between the topsheet 2 and the backsheet 3. Accordingly, both the end portions 9s, 9s of each elastic member 9 are fixed respectively between the topsheet 2 and the backsheet 3 via the panel members 10.

As illustrated in FIGs. 6 and 7, the diaper 1B includes the adhesive 12 which is discontinuously aligned in the longitudinal direction (Y-direction) and the width direction (X-direction) by applying the adhesive 12 to the plurality of elastic members 9 between the pair of panel members 10, 10 discontinuously in the longitudinal direction (Y-direction) and applying the adhesive 12 to the respective elastic members 9 discontinuously in the width direction (X-direction). As illustrated in FIG. 7, discontinuous adhesive 12 is applied between the topsheet 2 and each elastic member 9. Specifically, the discontinuous adhesive 12 is applied on a face of the topsheet 2 at the absorbent member 4 side, and is applied between the topsheet 2 and each elastic member 9. As illustrated in FIGs. 6 and 7, in the diaper 1B, the center section of each elastic member 9 is fixed to the topsheet 2 via the adhesive 12 which is discontinuously arranged in the width direction (X-direction) and which is discontinuously extended in the longitudinal direction (Y-direction). In other words, the center sections of the plurality of elastic members 9 located between the pair of panel members 10, 10 are fixed to the topsheet 2 via the adhesive 12 which is discontinuously arranged in the width direction (X-direction) and the longitudinal direction (Y-direction). Accordingly, the center sections of the plurality of elastic members 9 and the topsheet 2 are not fixed at regions where the adhesive 12 is not arranged. Since the center sections of the plurality of elastic members 9 located between the pair of panel members 10, 10 are fixed to the topsheet 2 via the adhesive 12 which is applied as described above, the gather is formed at the topsheet 2 while used owing to contraction of the plurality of elastic members 9 as illustrated in FIG. 7. As the adhesive 12, it is possible to adopt adhesive being similar to hot-melt adhesive for fixing the absorbent member 4 and the topsheet 2. Here, as a method of applying the adhesive 12, it is preferable to adopt an applying method with a comb gun to apply directly to the elastic members 9.

Materials for forming the disposable diaper 1B of the second embodiment will be described. The disposable diaper 1B of the second embodiment adopts materials being similar to the materials for forming the disposable diaper 1A of the first embodiment.

Operational effects obtained when using the abovementioned disposable diaper 1 B of the second embodiment of the present invention will be described.
Description of the effects of the disposable diaper 1B of the second embodiment will be performed on points which are different from the effects of the disposable diaper 1A of the first embodiment. Points which are not specifically described are similar to the effects of the disposable diaper 1A of the first embodiment and description of the effects of the disposable diaper 1A of the first embodiment is appropriately applied.

As illustrated in FIG. 7, since the gather is formed at the topsheet 2 owing to contraction of the plurality of elastic members 9 while used, the diaper 1B provides excellent appearance and an excellent texture.

Further, even contacting between the topsheet 2 and the laminated absorbent member 41 is caused by that a wearer lays on his/her back, or the like, the skin is contacted directly only to convex portions of the gather. Accordingly, fluid stuck to the skin can be suppressed to a small amount.

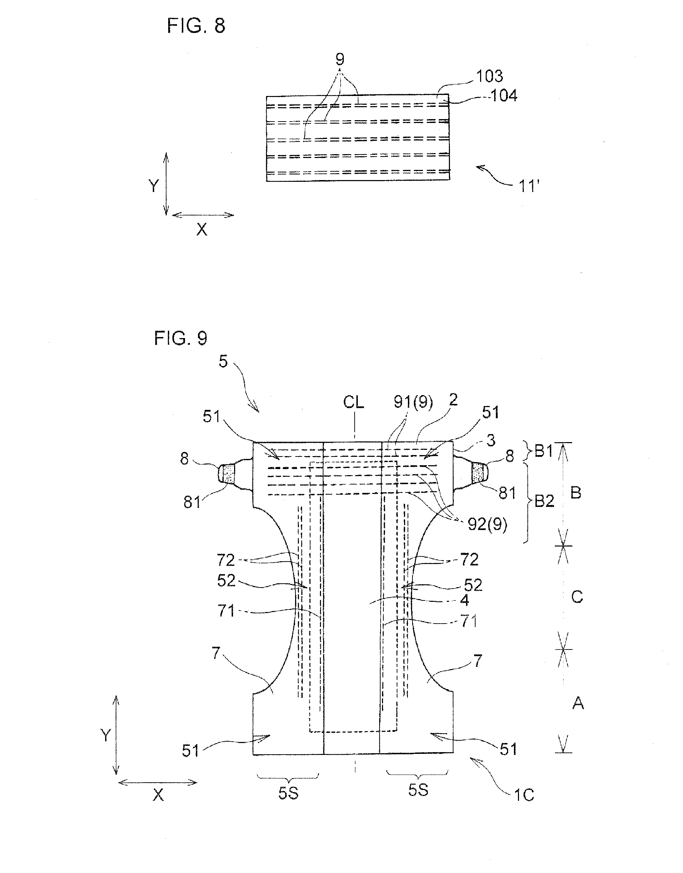

Next, a disposable diaper of a third embodiment of the present invention will be described based on FIG. 9.
A disposable diaper 1C (hereinafter, also called a diaper 1C) of the third embodiment will be described mainly on points being different from the disposable diaper 1A of the first embodiment. Points which are not specifically described are similar to the diaper 1A and description of the diaper 1A is appropriately applied.

As illustrated in FIG. 9, in the diaper 1C of the third embodiment, an absorbent body 5 has a shape elongated in the longitudinal direction (Y-direction) with both side edges in the longitudinal direction (Y-direction) narrowed inward at the center section in the longitudinal direction (Y-direction). Here, the width of each of both end sections in the longitudinal direction (Y-direction) is wider than the width at the center section in the longitudinal direction (Y-direction). As illustrated in FIG. 9, at both side sections 5s, 5s of the absorbent body 5 of the diaper 1C, side sheets 7 and a backsheet 3 extending outward in the width direction (X-direction) are joined respectively at the front section A and the rear section B to form side flap portions 51. As illustrated in FIG. 9, at the absorbent body 5 of the diaper 1C, the side sheets 7 and the backsheet 3 extending outward in the width direction (X-direction) are joined at the crotch section C to form leg flap portions 52.

As illustrated in FIG. 9, the rear section B of the diaper 1C includes a rear-waist section B 1 to which an absorbent member 4 is not placed and a below-waist section B2 to which the absorbent member 4 is placed. in the rear-waist section B1, a plurality of rear-waist stretchable members 91 being elastic members 9 is circumferentially arranged in parallel as being spaced in the longitudinal direction (Y-direction). Further, as illustrated in FIG. 9, in the rear section B of the diaper 1C, a plurality of torso stretchable members 92 being the elastic members 9 is circumferentially arranged in parallel as being spaced in the longitudinal direction (Y-direction) over a range of being 40% or more of the below-waist section B2 in the longitudinal direction (Y-direction) preferably, and in a range of 80% to 100% of the below-waist section B2 in the longitudinal direction (Y-direction) more preferably.

As described above, the side flap portions 51 are formed at the outer side of both the side edges of the both end sections in the longitudinal direction (Y-direction) of the absorbent body 5 of the diaper 1C. In the diaper 1C, the below-waist stretchable members 92 form a gather as being attached between the backsheet 3 which structures the side flap portion 51 and the side sheet 7 which structures the side flap portion 51 and is contacted to skin at the side flap portion 51 in the below-waist section B2. Specifically, as illustrated in FIG. 9, each end portion of the below-waist stretchable member 92 in a stretching state is extended to the side flap portion 51 in the below-waist section B2, and is fixed between the side sheet 7 and the backsheet 3. The side sheet 7 and the backsheet structure the side flap portion 51. Accordingly, a gather is formed at the side flap portion 51 in the below-waist section B2. Further, in the diaper 1C, as illustrated in FIG. 9, each end portion of the rear-waist stretchable member 91 in an extending state is extended to the side flap portion 51 in the rear-waist section B1 and is fixed between the backsheet 3 and the side sheet 7. The side sheet 7 and the backsheet 3 structure the side flap portion 51. Accordingly, a gather is also formed at the side flap portion 51 in the rear-waist section B1. The gather at the side flap portion 51 in the below-waist section B2 or the gather at the side flap portion 51 in the rear-waist section B 1 is formed by physically extending the below-waist stretchable member 92 or the rear-waist stretchable member 91.

The below-waist stretchable member 92 arranged at the below-waist section B2 has stretch stress which is smaller than that of the rear-waist stretchable member 91 arranged at the rear-waist section B1 preferably, and has a smaller sectional outer diameter than that of the rear-waist stretchable member 91 in addition to the stretch stress more preferably. Specifically, the below-waist stretchable member 92 is an elastic member having a thickness of 620 dtex or smaller preferably, and is an elastic member having a thickness in a range of 100 to 450 dtex more preferably. Meanwhile, the rear-waist stretchable member 91 is an elastic member having a thickness in a range of 300 to 950 dtex preferably.
It is preferable that the below-waist stretchable members 92 described above are arranged at the below-waist section B2 at intervals which are shorter than those in the longitudinal direction (Y-direction) of the rear-waist stretchable members 91 arranged at the rear-waist section B1. Specifically, the plurality of below-waist stretchable members 92 is circumferentially arranged in parallel at intervals of 7 mm or less in the longitudinal direction (Y-direction) preferably, and at intervals in a range of 3 to 6 mm more preferably. Meanwhile, the plurality of rear-waist stretchable members 91 is arranged at intervals in a range of 3 to 6 mm preferably in the longitudinal direction (Y-direction).

As illustrated in FIG. 9, the leg flap portions 52 are formed at the outer side of both the side edges at the crotch section C of the absorbent body 5 of the diaper 1C. At the leg flap portion 52, the leg gather is formed by arranging a stretchable material in the longitudinal direction and the standing gather which rises as having a fixed end at the side edge of the absorbent body 5 is formed. The leg gather and the standing gather are formed so that stretch and contraction do not substantially appear at a stretch-contraction section. Specifically, an elastic member 72 for forming the leg gather is strongly fixed to the leg flap portion 52 with adhesive in a stretching state in the longitudinal direction (Y-direction). The leg gather is formed while worn owing to contraction force of the elastic member so that the leg gather does not substantially provide stretch and contraction at the stretch-contraction section. Further, an elastic member 71 for forming the standing gather is strongly fixed to a vicinity of a free end of an end portion at the inner side (being close to the center line CL) of each side sheet 7 in the X-direction with adhesive in a stretching state in the longitudinal direction (Y-direction). The standing gather is formed as a section having a predetermined width from the free end is separated from the topsheet 2 while worn owing to a contraction force of the elastic member so that the standing gather does not substantially provide stretch and contraction at the stretch-contraction section.

Materials for forming the disposable diaper 1C of the third embodiment will be described. The disposable diaper 1C of the third embodiment adopts materials being similar to the materials for forming the disposable diaper 1A of the first embodiment.

Operational effects obtained when using the abovementioned disposable diaper 1C of the third embodiment of the present invention will be described.
Description of the effects of the disposable diaper 1C of the third embodiment will be performed on points which are different from the effects of the disposable diaper 1A of the first embodiment. Points which are not specifically described are similar to the effects of the disposable diaper 1A of the first embodiment and description of the effects of the disposable diaper 1A of the first embodiment is appropriately applied.

As illustrated in FIG. 9, in the diaper 1C, both end portions 9s, 9s of the elastic members 9 fixed to the side sheet 7 and the backsheet 3 can be formed at the side flap portions 51 extending outward in the width direction (X-direction). Accordingly, the distance of separation between the absorbent member 4 and the topsheet 2 illustrated in FIGs. 3 and 7 is enlarged, so that body fluid once absorbed by the absorbent member is less likely to return.

Not limited to the abovementioned disposable diapers 1A, 1B, and 1C of the first to third embodiments, the disposable diaper of the present invention may be appropriately modified. Further, structural elements in the abovementioned disposable diapers 1A, 1B, and 1C of the first to third embodiments may be appropriately combined without departing from the spirit of the present invention.

For example, in the abovementioned disposable diapers 1A, 1B, and 2C of the first to third embodiments, both the end portions 9s, 9s of the elastic members 9 are fixed between the topsheet 2 and the backsheet 3 via the pair of panel members 10, 10. However, both the end portions 9s, 9s of the elastic members 9 may be fixed directly between the topsheet 2 and the backsheet 3 without using the pair of panel members 10, 10. Further, in the abovementioned disposable diapers 1A, 1B of the first and second embodiments, the ladder-shaped elastic structure 11 (see FIG. 4) in which the plurality of elastic members 9 is arranged between the pair of panel members 10, 10 as being spaced in the longitudinal direction (Y-direction) is arranged on the skin-facing face of the laminated absorbent member 41 located at the rear section B, as illustrated in FIGs. 1 and 6. However, instead of the ladder-shaped elastic structure 11, a rectangle-shaped elastic structure 11' which is formed by joining two sheets 103, 104 which are rectangle-shaped as being elongated in the X-direction with joining means (adhesive) such as hot-melt type adhesive in a state that a plurality of elastic members 9 is arranged at intervals in the longitudinal direction (Y-direction) as being sandwiched between the two sheets 103, 104 may be arranged on the skin-facing face of the laminated absorbent member 41 located at the rear section B, as illustrated in FIG. 8. The two sheets 103, 104 may be formed of the same material as the sheet pieces 101, 102 which form the elastic structure 11.

The disposable diaper of the present invention may be a disposable diaper for an infant or an adult. In relation to the abovementioned embodiment, additional subjects (disposable diapers) are further disclosed in the following.

[1] A disposable diaper comprising an elongated absorbent body which includes a liquid permeable topsheet, a liquid low-permeable backsheet, and an absorbent member arranged between the sheets, and which is sectioned into a front section, a rear section, and a crotch section, wherein
the absorbent member includes a laminated absorbent member which is formed by sandwiching high-absorbent polymer with two sheets of nonwoven and which is arranged over a range from the front section to the rear section,
the absorbent member and the topsheet are not fixed at the rear section and are fixed at the front section and the crotch section,
the absorbent body includes a plurality of elastic members across the absorbent member in a stretching state between the absorbent member and the topsheet at the rear section, and
the plurality of elastic members is arranged as being mutually spaced in the longitudinal direction while both end portions of each elastic member are fixed between the topsheet and the backsheet, so as to separate the absorbent member from the topsheet owing to contraction of the plurality of elastic members while used.
2. The disposable diaper according to claim 1, wherein the both end portions of each of the plurality of elastic members are fixed with a pair of panel members extending in the longitudinal direction and are fixed between the topsheet and the backsheet via the pair of panel members.

[2] The disposable diaper according to subject [1], wherein the both end portions of each of the plurality of elastic members are fixed with a pair of panel members extending in the longitudinal direction and are fixed between the topsheet and the backsheet via the pair of panel members.
[3] The disposable diaper according to subject [1] or [2], further including a right-left pair of stretchable panels arranged at both bilateral outer sides of the rear section of the absorbent body, wherein the plurality of elastic members is arranged as being spaced in the longitudinal direction with a part of the elastic members located beyond a section which is sandwiched by the pair of stretchable panels.

[4] The disposable,diaper according to any one of subjects [1] to [3], wherein each of the elastic members is arranged as being equally spaced in the longitudinal direction.
[5] The disposable diaper according to any one of subjects [1] to [3], wherein each of the elastic members are arranged without being equally spaced in the longitudinal direction.
[6] The disposable diaper according to any one of subjects [1] to [5], wherein both end portions of each of the elastic members are fixed respectively at the outer side from a side edge of the absorbent member.
[7] The disposable diaper according to any one of subjects [1] to [6], wherein an elastic structure is formed by fixing both end portions of each of the plurality of elastic members with a pair of panel members extending in the longitudinal direction and the elastic structure is arranged between the absorbent member and the topsheet.
[8] The disposable diaper according to any one of subjects [2] to [7], wherein the pair of panel members are fixed between the topsheet and the backsheet and the elastic members are not fixed to the absorbent member.
[9] The disposable diaper according to any one of subjects [1] to [8], wherein adhesive to fix the top sheet and the elastic member is applied on a face of the topsheet at the absorbent member side.

[10] The disposable diaper according to subject [9], wherein the adhesive is discontinuously aligned in the longitudinal direction and the width direction by applying the adhesive to the elastic members in the longitudinal direction and applying the adhesive to the elastic members discontinuously in the width direction.
[11] The disposable diaper according to subject [9] or [10], wherein the adhesive is applied between the topsheet and the respective elastic members.
[12] The disposable diaper according to any one of subjects [9] to [11], wherein a center section of each of the elastic members is fixed to the topsheet via the adhesive which is discontinuously arranged in the width direction and which is discontinuously extended in the longitudinal direction, and a gather is formed at the topsheet owing to contraction of the elastic members while used.
[13] The disposable diaper according to any one of subjects [1] to [12], wherein the topsheet has an uneven shape.

[14] The disposable diaper according to any one of subjects [1] to [13], wherein the absorbent member includes a sublayer which is fixed on the laminated absorbent member located at the crotch section.
[15] The disposable diaper according to subject [14], wherein the sublayer has area being equal to or less than 100% of total area of skin-facing face of the laminated absorbent member.
[16] The disposable diaper according to subject [14] or subject [15], wherein a basis weight of the sublayer is in a range of 40 to 300 g/m².
[17] The disposable diaper according to any one of subjects [14] to [16], wherein a sheet structured mainly with hydrophilic fibers is used as the sublayer.
[18] The disposable diaper according to any one of subjects [14] to [17], wherein a sheet with content of hydrophilic fibers being 90% or more by mass is used as the sublayer.

[19] The disposable diaper according to any one of subjects [1] to [18], wherein the rear section includes a rear-waist section to which the absorbent member is not placed and a below-waist section to which the absorbent member is placed, and a plurality of rear-waist stretchable members being the elastic members is circumferentially arranged at the rear-waist section in parallel as being spaced.
[20] The disposable diaper according to subject [19], wherein a plurality of below-waist stretchable members being the elastic members having smaller stretch stress than the rear-waist stretchable members are circumferentially arranged in parallel as being spaced over a longitudinal range of being 40% or more of the below-waist section.
[21] The disposable diaper according to subject [19] or subject [20], wherein side flap portions are formed at the outer side of both side edges of both end sections in the longitudinal direction of the absorbent body, and the below-waist stretchable members form a gather as being attached between the backsheet which structures the side flap portion and a sheet which is contacted to skin at the side flap portion in the below-waist section.
[22] The disposable diaper according to any one of subjects [19] to [21], wherein the plurality of below-waist stretchable members with thickness of 620 dtex or smaller having smaller stretch stress and a sectional outer diameter than those of the rear-waist stretchable members is circumferentially arranged at the below-waist section in parallel at intervals of 7 mm or less being shorter than intervals of the rear-waist stretchable members.
[23] The disposable diaper according to any one of subjects [19] to [22], wherein leg flap portions are formed at the outer side of both side edges at the crotch section of the absorbent body, and a leg gather is formed at the leg flap portions by arranging a stretchable material in the longitudinal direction and a standing gather which rises as having a fixed end at a side edge of the absorbent body is formed, so as to form the leg gather and the standing gather without substantially appearing stretch and contraction at a stretch-contraction section.
[24] The disposable diaper according to any one of subjects [21] to [23], wherein the gather at the side flap portion in the below-waist section is formed by physically extending the below-waist stretchable member.
[25] The disposable diaper according to any one of subjects [1] to [23], further including a fastening tape to which a fastening portion is attached.

### Examples

In the hollowing, the present invention will be described more specifically with examples. Here, the present invention is not limited to such examples.

### [Example 1]

An open-style disposable diaper for a baby (Pampers (a registered trademark) Cruisers with Dry Max, size 4, manufactured by The Procter & Gamble Company) was used as a base sample. At a rear section of the base sample, adhesive was solidified by spraying cold spray on a top material and the top material was peeled off from an absorbent body. Then, an urethane-made thread-like elastic members being 500 dtex were arranged thereat across the width direction as being stretched to be twice of a natural length. Two strips of the elastic members were arranged for one location with an interval of 5 mm. Specifically, arranging of the above was performed at four locations in total being a position distanced from a rear section end part of an intermediate layer by 10mm, and positions distanced therefrom by 40 mm, 80 mm and 120 mm. Subsequently, the peeled top material was placed on the absorbent member and a gather again, and end portions thereof in the width direction and the longitudinal direction were bonded. Thus, a sample for Example 1 was obtained. Here, an adherence property of the once-solidified adhesive did not appear again, so that the top material and the absorbent member were separated owing to contraction of thread rubber.

### [Example 2]

Using hydrophilic polypropylene spunbond nonwoven with a basis weight being 17 g/m² instead of a top material, four sides of the nonwoven were bonded respectively to a backsheet of a base sample after the top material of the base sample was peeled off from an absorbent member with a similar method to Example 1. Here, adhesive was not applied at all to a section which overlapped with an absorbent body of the nonwoven. Other than the above, an open-style disposable diaper was prepared by arranging and bonding urethane-made thread-like elastic members as being similar to Example 1. Thus, a sample for Example 2 was obtained.

### [Example 3]

After a top material of a base sample was peeled off from an absorbent member with a similar method to Example 1, air-through nonwoven structured with hydrophilic polypropylene-polyethylene core-clad fibers being 2 dtex having a basis weight of 25 g_{/}m² was used instead of the top material. Other than the above, an open-style disposable diaper was prepared by arranging and bonding urethane-made thread-like elastic members as being similar to Example 2. Thus, a sample for Example 3 was obtained.

### [Example 4]

After a top material of a base sample is peeled off from an absorbent member with a similar method to Example 1, air-through nonwoven structured with hydrophilic polypropylene-polyethylene core-clad fibers being 2dtex having a basis weight of 25 g/m² and having an uneven shape with a thickness between apexes of a top face and a back face being about 3.5 mm was used instead of the top material. Other than the above, an open-style disposable diaper was prepared by arranging and bonding urethane-made thread-like elastic members as being similar to Example 2. Thus, a sample for Example 4 was obtained.

### [Comparative Example 1]

A base sample was used as a sample for comparison Example 1. Here, a top material was made of spunbond nonwoven.

### [Evaluation]

Liquid return amounts were measured with a method described below using samples (disposable diapers) of the Examples and the comparison Example. The result is indicated in Table 1. The extent of the liquid return amount is closely related to absorption performance of a disposable diaper, especially, to a dry feeling at a skin-facing face (topsheet). The less liquid return amount provides higher rating with higher absorption performance of a disposable diaper as being superior in dry feeling.

### <Method of measuring liquid return amount>

A disposable diaper for a baby was horizontally placed with a topsheet faced upward and artificial urine of 40 g was poured thereto. Pouring was performed to a center part in the width direction of an absorbent member at a rear section where a sublayer was not arranged. After leaving as it is for five minutes, sixteen sheets of filter paper (hard filter paper ADVANTEC (registered trademark) 5C manufactured by Toyo Roshi Kaisha, LTD.) of 10 centimeters square were overlapped and placed on the poured position. Then, pressurization was performed for two minutes with a load of 0.7 kPa or 0.1 kPa. Thus, artificial urine liquid-returned with pressurization was absorbed by the filter paper. A weight of the filter paper increased with the absorption was measured and difference against a weight of the filter paper before the absorption was taken as the liquid return amount.

**[Table 1]**

| | Unit | Example 1 | Example 2 | Example 3 | Example 4 | Comparative Example 1 |
|---|---|---|---|---|---|---|
| Liquid return amount | g | 0.07 | 0.12 | 0.03 | 0.13 | 2.44 |
| Load below 0.1 kPa | | | | | | |
| Liquid return amount | g | 0.15 | 0.56 | 0.57 | 0.13 | 3.94 |
| Load of 0.7 kPa | | | | | | |

As clearly seen from the result indicated in Table 1, it is revealed that the disposable diapers of the examples (the present invention) have less liquid return amounts in both cases of the load at measuring the liquid return amount being 0.7 kPa and being below 0.1 kPa. The load of 0.7 kPa corresponds to a load applied to a diaper in a state that a young month-old infant wearing the diaper lies on his/her back. When the liquid return amount is below 2g at the load of 0.7 kPa, liquid is not moved along a skin in a case that liquid exists between a skin of a wearer and the topsheet. Further, the load below 0.1 kPa reflects a feeling of touching a surface (skin-facing face) of a diaper without substantially little pressurization to the diaper. When the liquid amount is below 0.2 g at the load of 0.1 kPa, a wearer hardly feels wet.

Effects of the present invention were tested at a rear section without having, a sublayer. It is supposed to have a state that a large amount of urine is discharged and that the urine is spread to the entire face of an absorbent member. However, for spreading artificial urine from an urination section to the rear section, a large amount of artificial urine is to be required and influence of a variety of variations for respective samples is hard to avoid. For convenience sake, artificial urine of 40 g approximately being an amount of one time of urination of an infant was poured to the rear section.
The top material of Example 1 was in a state that adhesive originated from the base sample (Pampers (the registered trademark)) was attached. On the other hand, in Examples 2 to 4, adhesive was not attached at all to the top material. In general, adhesive used for a disposable diaper was hydrophobic. It is revealed that staying of adhesive on a surface of the top material at the absorbent member side is preferable to suppress returning of urine in the absorbent member to a diaper top surface.
It is revealed that a thicker top material is preferable to have higher effect of preventing skin wet while separating the absorbent and a wearer's skin in distance. Further, it is revealed that a top material having a smaller basis weight and larger thickness is preferable to lessen a fear that urine is absorbed from the absorbent body to a skin with capillary phenomenon. In particular, it is revealed that a top material having an uneven shape is more preferable to lessen contact area with a skin and to suppress transmission of a wet feeling.

### Industrial Applicability

According to the disposable diaper of the present invention, body fluid once absorbed by an absorbent member is less likely to return while increasing absorbing speed of body fluid.

## Claims

1. A disposable diaper comprising an elongated absorbent body which includes a liquid permeable topsheet, a liquid low-permeable backsheet, and an absorbent member arranged between the sheets, and which is sectioned into a front section, a rear section, and a crotch section, wherein
the absorbent member includes a laminated absorbent member which is formed by sandwiching high-absorbent polymer with two sheets of nonwoven and which is arranged over a range from the front section to the rear section,
the absorbent member and the topsheet are not fixed at the rear section and are fixed at the front section and the crotch section,
the absorbent body includes a plurality of elastic members across the absorbent member in a stretching state between the absorbent member and the topsheet at the rear section, and
the plurality of elastic members is arranged as being mutually spaced in the longitudinal direction while both end portions of each elastic member are fixed between the topsheet and the backsheet, so as to separate the absorbent member from the topsheet owing to contraction of the plurality of elastic members while used.

2. The disposable diaper according to claim 1, wherein the both end portions of each of the plurality of elastic members are fixed with a pair of panel members extending in the longitudinal direction and are fixed between the topsheet and the backsheet via the pair of panel members.

3. The disposable diaper according to claim 1 or claim 2, further comprising a right-left pair of stretchable panels arranged at both bilateral outer sides of the rear section of the absorbent body, wherein the plurality of elastic members is arranged as being spaced in the longitudinal direction with a part of the elastic members located beyond a section which is sandwiched by the pair of stretchable panels.

4. The disposable diaper according to any one of claims 1 to 3, wherein adhesive to fix the topsheet and the elastic member is applied on a face of the topsheet at the absorbent member side.

5. The disposable diaper according to any one of claims 1 to 4, wherein the topsheet has an uneven shape.

6. The disposable diaper according to any one of claims 1 to 5, wherein center sections of the plurality of elastic members are fixed to the topsheet via adhesive which is discontinuously arranged in the width direction and is discontinuously extended in the longitudinal direction, so as to form a gather at the topsheet owing to contraction of the plurality of elastic members while used.

7. The disposable diaper according to any one of claims 1 to 6, wherein the absorbent member includes a sublayer which is fixed on the laminated absorbent member located at the crotch section.

8. The disposable diaper according to any one of claims 1 to 7, wherein
the rear section includes a rear-waist section to which the absorbent member is not placed and a below-waist section to which the absorbent member is placed, and
a plurality of rear-waist stretchable members being the elastic members is circumferentially arranged at the rear-waist section in parallel as being spaced.

9. The disposable diaper according to claim 8, wherein a plurality of below-waist stretchable members being the elastic members having smaller stretch stress than the rear-waist stretchable members are circumferentially arranged in parallel as being spaced over a longitudinal range of being 40% or more of the below-waist section.

10. The disposable diaper according to claim 8 or claim 9, wherein
side flap portions are formed at the outer side of both side edges of both end sections in the longitudinal direction of the absorbent body, and
the below-waist stretchable members form a gather as being attached between the backsheet which structures the side flap portion and a sheet which is contacted to skin at the side flap portion in the below-waist section.

11. The disposable diaper according to any one of claims 8 to 10, wherein the plurality of below-waist stretchable members with thickness of 620 dtex or smaller having smaller stretch stress and a sectional outer diameter than those of the rear-waist stretchable members is circumferentially arranged at the below-waist section in parallel at intervals of 7 mm or less being shorter than intervals of the rear-waist stretchable members.

12. The disposable diaper according to any one of claims 8 to 11, wherein
leg flap portions are formed at the outer side of both side edges at the crotch section of the absorbent body, and
a leg gather is formed at the leg flap portions by arranging a stretchable material in the longitudinal direction and a standing gather which rises as having a fixed end at a side edge of the absorbent body is formed, so as to firm the leg gather and the standing gather without substantially appearing stretch and contraction at a stretch-contraction section.

13. The disposable diaper according to any one of claims 10 to 12, wherein the gather at the side flap portion in the below-waist section is formed by physically extending the below-waist stretchable member.
